(19) Europäisches Patentamt
European Patent Office
Office européen des brevets

(11) EP 0 751 225 B1

(12) **EUROPEAN PATENT SPECIFICATION**

(45) Date of publication and mention of the grant of the patent:
**28.03.2001 Bulletin 2001/13**

(51) Int Cl.[7]: **C12Q 1/37**

(21) Application number: **96114931.7**

(22) Date of filing: **14.10.1991**

(54) **Process for the identification of proteolytic activities and/or inhibitors thereof**

Verfahren zur Feststellung von proteolitischen Wirkungen und/oder deren Inhibitoren

Procédé pour l'identification d'activités protéolytiques et/ou des inhibiteurs de celles-ci

(84) Designated Contracting States:
**AT BE CH DE DK ES FR GB GR IT LI LU NL SE**

(30) Priority: **15.10.1990 IT 4836590**
**15.04.1991 IT RM910261**

(43) Date of publication of application:
**02.01.1997 Bulletin 1997/01**

(62) Document number(s) of the earlier application(s) in accordance with Art. 76 EPC:
**91830428.8 / 0 481 930**

(73) Proprietor: **TECNOGEN S.C.P.A.**
**81015 Piana de Monte Verna (Caserta) (IT)**

(72) Inventors:
- **Fassina, Giorgio**
**20131 Milano (IT)**
- **Corti, Angelo**
**20131 Milano (IT)**

(74) Representative: **de Simone, Domenico et al**
**Ing. Barzanò & Zanardo Roma S.p.A.**
**Via Piemonte 26**
**00187 Roma (IT)**

(56) References cited:
**EP-A- 0 056 015**
**EP-A- 0 080 279**
**EP-A- 0 168 738**
**EP-A- 0 255 341**
**EP-A- 0 318 318**
**EP-A- 0 325 472**
**EP-A- 0 411 503**
**EP-A- 0 428 000**
**WO-A-90/00252**

- **PROCEEDINGS OF THE NATIONAL ACADEMY OF SCIENCES OF USA, vol. 85, no. 15, August 1988, WASHINGTON US, pages 5409-5413, XP002016804 J P TAM: "Synthetic peptide vaccine design: synthesis and properties of a high-density multiple antigenic peptide system"**
- **PEPTIDES, CHEMISTRY AND BIOLOGY. PROC. XTH AMERICAN PEPTIDE SYMPOSIUM, MAY 23-28, S. LOUIS, MISSOURI, USA, 1988, ESCOM, LEIDEN, pages 354-363, XP002016805 I CHAIKEN ET AL.: "Sequence simplification and randomization and the design of the peptide recognition surface"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 751 225 B1

Printed by Jouve, 75001 PARIS (FR)

## Description

This invention relates to a process for the identification of proteolytic activities and/or of inhibitors thereof in biological fluids, fermentation broths, conditioned culture soils, cell extracts, plant extracts.

The hypothesis that interaction sites between pairs of interacting proteins are coded by complementary DNA sequences has been first formulated by Biro (Biro, Medical Hypothesis 7:669, 1981) and then confirmed experimentally by Blalock et al. (Bost et al., PNAS 82: 1372, 1985). The latters have shown a meaningful hydropathic complementarity between amino acids deduced from complementary codons (Blalock and Smith, BBRC, 121:203, 1984), read in the 3'-5' or the 5'-3' directions (Elalock and Bost, Biochem. J., 234:679, 1986). The initial observations have then been confirmed experimentally in a large number of other systems of biological importance, both allowing the ex novo design to be carried out of peptides capable of associating target peptides or proteins, as is the case of fibronectin (Brentani et al., PNAS, 85:364, 1988), insulin (Knutson, J. Biol. Chem., 263:14146, 1988), Arg8-vasopressing (Fassina et al., Biochemistry, 28:8811, 1989), the substance P (Bost and Blalock, Methods in Enzymology, 168:16, 1989), ribo-nuclease peptide S (Shai et al., Biochemistry, 28:8804, 1989), the C-raf protein (Fassina et al., J. Biol. Chem., 264:11252, 1989), and allowing the prediction of the contact sites between proteins and their receptors to be performed as well, like in the case of interleukin 2, of transferring and of the EGF or Epidermal Growth Factor (Bost et al., BBRC 28:1375, 1985).

Recently the authors of this invention have realized (Italian Patent Application No. 21396 A/89 and No. 20755 A/90) a computer-aided procedure that allows peptide chains hydropathically complementary to be obtained capable of interacting with given amino acid sequences, employing just primary sequence information of said sequences. The peptides so obtained show to have a higher binding affinity with respect to those obtained from complementary DNA, and they can be employed advantageously in diagnostic and analytical procedures, which are often carried out in the solid phase.

The methods already known for the immobilization of low molecular weight peptides (M.W. 1000-4000) on solid supports can be essentially divided into the methods that are based on a covalent interaction between the peptide and the support, and the methods in which the peptide is linked or adsorbed in a non-covalent way. In the first instance, generally the chemical reactivity of the amino or carboxyl groups of the peptides is exploited for esterification reactions with suitable groups which are present on the solid support. As quite often the peptide of interest can contain more than one reactive group (for instance, amino or carboxyl groups), the immobilization can be hardly selected towards a particular group, so being non-homogeneous and unforeseeable. In the case in which the peptide has a single functional group, the immobilization turns out to be oriented, but the closeness to the solid phase quite often causes a partial or total loss of the initial recognition capabilities. The introduction of suitable spacers between the solid phase and the peptide obviates some drawbacks of such kind, but such approach considerably increases the cost of the support or of the peptide, considerably decreases the total capability, and in addition it is to be adjusted in its details for each peptide individually, and introduces some changes which are due to non-specific interactions between the spacer and the other molecules. The immobilization of peptides by means of non-specific interactions on solid supports is also illustrated in the literature (Geerligs et al., J. Immunol. Methods, 106:239, 1988). In that case, the orientation of the peptide is fully undeterminable, because many residues are involved in the interaction in an unforeseeable way.

Moreover, the same authors have found a remarkable limitation to the employment of hydropathically complementary peptides which are the object of the Italian Patent Application No. 20755 A/90, because of the loss or reduction of affinity when, employing the methods disclosed, they are immobilized on a solid phase, being it a solid support previously derivatized for preparing an affinity chromatographic column, or a plastic plate which has been suitably adjusted to carry out analytical tests.

Recently (Tam, Proc. Natl. Acad. Sci. USA 85:5409, 1988) a procedure has been realized for the production of antipeptide antibodies employing multiple peptides as antigens, said peptides being covalently linked to an octadentate polylysine nucleus. The resulting molecules shows an increased immunogenic activity, up to a degree much higher than that of the same linear peptides.

The authors of the present invention have surprisingly found that the covalent or non-covalent immobilization on solid supports of peptides which are hydropathically complementary to known peptides, and are synthesized in a multiple and non linear form according to a process similar to Tam's procedure (cf. above) for the production of antigenic multiple peptides, allows many of the drawbacks described previously to be obviated. Indeed, during the immobilization process, just some of the peptide chains of the multimer molecule are statistically involved in the interaction with the solid phase, whereas the other ones show fully accessible and properly oriented to carry out the recognition with hydropathically complementary peptide sequences. Accordingly, it is possible to employ the affinity of hydropathically complementary peptides in diagnostic tests and in analytical and preparative methodologies which make use of affinity chromatographic partitions and, in general, in the field of immobilization of the peptide ligands. In particular, hydropathically complementary peptides can be employed for identifying reacting activities with the same. Indeed, in the industrial pharmaceutical field, the search for new, pharmacologically active molecules is carried out through the study

of the biological activities of fermentation broth, of conditioned culture soils, of cell extracts, of plant extracts and of other products of fermentation origin containing a large number of bioorganic molecules which are uncharacterized and are endowed with activities of the most different kinds.

The peptides and the proteins, which comprise also those having hormonal actions, endowed with pharmacological activities and which, while biologically synthesized originally in the form of high molecular weight precursors, require activation by means of sequence-specific proteolytic enzymes, are numerous. Accordingly, in the pharmacological field the research of both such enzymes and of molecules capable of inhibiting such conversions for contrasting the effect of the same is very important.

EP 325472 at claim 5 discloses a method where the enzymatic activity liberates an enhancer which increases the oxidation of a chromogen substrate. Therefore the measure is performed on the amount of liberated enhancer.

EP 318318 at claim 1 discloses a process wherein the analyte may be hydrolysed by elastase to form a compound, and such compound is to be measured.

EP 168738 at claim 1 discloses a method wherein the protease produce a detectable compound.

EP 056015 discloses compounds acting as chromogenic substrates for proteolytic enzymes, wherein the formation of a detectable chromophore may be measured further to the proteolytic attack.

EP 428000 describes a method wherein again the formation of the cleaved product is measured, and not the disappearance of substrate as in the instant application.

EP 411503 discloses a method for designing peptides able to bind to selected amino acid sequences of peptides or proteins. The application of such peptides for assays for detecting proteolytic activities is not disclosed, nor even suggested.

Generally, the search for enzymes and consequently for their specific inhibitors is made easier by the availability of chromogenic synthetic substrates that, mimicking the natural substrate, allow the enzymatic activity of biological extracts not necessarily purified to be monitored, as well as the effect of molecules having a possible inhibiting activity. However, in many cases, said synthetic substrates are not available and the enzymatic conversion reactions are to be followed directly on the natural substrate, with remarkable problems connected quite often to the low detection limits obtainable and to the complexity of the tests which can be employed. Anyway, even in the case of chromogenic substrates, the tests for detecting proteolytic activities or the presence of inhibitors show to be hardly adaptable to the rapid, convenient and sensitive procedures in the solid phase, which are increasingly employed in the diagnostic field, but up to the present time are not exploited for tests on protease activities or on activities which inhibit the same.

Recently a 21-amino acid peptide has been identified and characterized, which has been called "endothelin" (El) and is endowed with a powerful vasoconstricting action (Yanagisawa et al., 1988, Nature 332: 411-415). Endothelin is synthesized by the endothelial cells in the form of a precursor having 200 amino acids (prepro-endothelin), first converted into pro-endothelin (38 amino acids) and then into mature endothelin (1-21) through proteolytic degradation by means of one or more specific enzymes which have not been characterized as yet and are called "ECE" (endothelin converting enzymes). Pro-endothelin is pharmacologically inactive, but it can be activated both in vitro and in vivo, also by the action of wide-spectrum proteolytic enzymes like chymotrypsin and cathepsin. Moreover, it has also been shown that the presence of protease inhibitors like phosphoramidone not only prevent pro-endothelin from being converted into endothelin, but also prevents the blood pressure from increasing in rats when pro-endothelin is administered to the same through injection. It is of remarkable importance that such inhibitor gradually reduces also the blood pressure in rats suffering from spontaneous hyper-tension (McMahon et al., 1990, Proc. 2nd Internat. Meeting on Endothelin, Tsukuba City, Japan).

ECE chromogenic substrates for employment in the identification of such proteolytic activities or of similar proteolytic activities as well as of inhibitors of said activities are not available at present.

Accordingly, it is clearly evident there is the need for a rapid, sensitive, automatizable process that can be easily carried out for the identification and characterization of proteolytic activities or of activities capable of inhibiting the same, in biological fluids, fermentation broth, conditioned culture soils, cell extracts, plant extracts, which process, in particular, can be employed for the research of ECE inhibitors more specific and more active than those already available, for obtaining new molecules having hypotensive activity, as well as for the isolation and the characterization of ECE itself, in order to design and synthesize in vitro synthetic molecules having an inhibiting activity of the proteolytic activities.

According to this invention, the authors of the same have surprisingly found that also the pro-endothelin 16-32 fragment undergoes a proteolytic degradation at the level of the 21-22 residues, because of the action of enzymes like chymotrypsin, the kinetic characteristics being similar to those observed with the whole pro-endothelin. The 8 Δ ET (16-29) molecule, both in solution and in the solid phase, recognizes the fragment 16-32 of pro-endothelin, even though it has been derivatized with biotin, whereas it is uncapable of associating the fragments 16-21 and/or 22-32 obtained by chymotryptic degradation. Accordingly, it has been possible to exploit the immobilization of the 8 Δ ET (16-29) molecule on plastic plates in order to design a solid-phase test for detecting the derivatized biotin ET (16-32). Such test allows many of the drawbacks previously met with in the search for proteolytic enzymes to be obviated, in particular

with specific activities towards pro-ET and of activities inhibiting the same.

The object of the present invention a process for the identification of proteolytic activities and/or of inhibitors of such activities, said process comprising the reaction

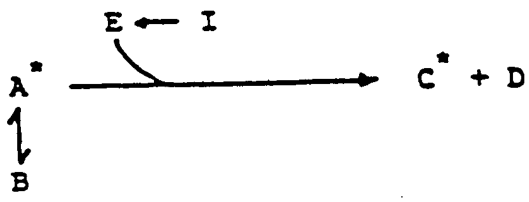


wherein E represents said proteolytic activity, capable of catalyzing the conversion of a substrate A comprising at least one protein fragment labelled with at least one site of proteolytic attack, to the fragments C and D,
I represents said inhibitors of such conversion, B represents a ligand which is capable of recognizing and bonding said substrate A but not said fragments C and D; the asterisk pointing out that said substrate A is labelled.

According to the invention it is provided a process for the identification of a proteolytic activity (E) and/or of an inhibitor (I) of such activity (E), in a biological material, said process comprising:

a) incubating a substrate A, comprising at least a labelled protein fragment having at least a proteolytic attack site for said proteolytic activity (E) with said biological material;
b) allowing the reaction to convert the substrate (A) into fragments (C) and (D);
c) measuring the amount of substrate A by means of a ligand (B) which is capable of recognizing said substrate (A) but not said fragments (C) and (D).

According to the present invention, said fragment is labelled by means of radioisotopes, or of enzymes, or of fluorescent compounds or of chromophores, or of biotin.

Preferably, said fragment is labelled with biotin and can be evidenced by incubation with streptavidin conjugated with peroxidase.

Again according to this invention, the process comprising means for the separation of the protein fragment-ligand complex from unlinked protein fragments and from the proteolysed derivatives.

Said separation means are conveniently realized through the immobilization on a solid phase of said ligand, and washing off said protein fragments which are not bonded and said proteolysed derivatives.

Again according to the present invention, said solid phase comprises plastic plates divided into compartments.

Again according to this invention, the process comprises the step of measuring said labelled protein fragments linked to said ligand immobilized on a solid phase.

Again it is an object of the present invention a process wherein said protein substrate comprises a fragment of the pro-endothelin and said ligand comprises amino acid sequences which are idropathically complementary to said fragment of the pro-endothelin.

Again more preferably, said fragment of the pro-endothelin is made up of the amino acid 16 up to the amino acid 32 of the formula HLDIIWVNTPEHIVPYG, and said detection molecule comprises the molecule 8 Δ ET (16-29).

According to the present invention, said proteolytic attack site is comprised between the amino acids 21-22 of the fragment 16-32 of pro-endothelin, in such a way as to give rise, if in the presence of proteolytic activities, to two fragments ET (16-21) and ET (22-32) which do not become linked to said molecule 8 Δ ET (16-29).

The presence of proteolytic activities suitable to convert said substrate ET (16-32) to ET (16-21) and to ET (22-32) is easily put into evidence, through the incubation of suitable amounts of said biotin-derivatized substrate, with the sample under test, and determining the amount of biotinized derivative linked to the plates. In a similar way, the presence of inhibitors of the conversion reaction is put into evidence through the incubation of suitable amounts of biotinized derivative and chymotrypsin solutions or raw preparations of ECE, with the sample under test, and then determining the amount of said biotinized substrate linked to the plate.

This invention will be disclosed in the following just for illustrative and not for limitative purposes by means of the following application examples, wherein the following figures will be referred to, wherein:

Figure 1A represents the structure of the polylysine nucleus employed for the synthesis of 8 Δ ET (16-29);
Figure 1B represents the structure of the nonlinear peptide 8 Δ ET (16-29);
Figure 1C represents the structure of the peptide ET (16-29);

Figure 1D represents the structure of the nonlinear peptide 8ET (16-29);
Figure 2 illustrates the different performance of the columns prepared with Δ ET (16-29) and 8 Δ ET (16-29);
Figure 3A illustrates the purification of the biotinylated derivative of ET (1-32) on an affinity column prepared with the peptide 8 Δ ET (16-29);
Figure 3B illustrates am analysis carried out by means of RP-HPLC of the biotin ET (1-32) product purified through affinity chromatography;
Figure 4 illustrates the binding of the biotinylated derivative of ET (1-32) with plastic microcontainers derivatized with 8 Δ ET (16-29);
Figure 5 illustrates the interaction specificity between ET (1-32) and 8 Δ ET (16-29) immobilized on plastic microcontainers;
Figure 6 illustrates the various affinity of the peptides ET (16-29) (A) and 8ET (16-29) (B) towards 8 Δ ET (16-29);
Figure 7A illustrates the structure of the polylysine nucleus employed for the synthesis of 8 Δ TNF (144-157);
Figure 7B illustrates the structure of the peptide 8 Δ TNF (144-157);
Figure 8A represents the binding of TNFα with an affinity column prepared with the peptide 8 Δ TNF(144-157);
Figure 8B represents an analysis carried out through RP-HPLC of the TNFα purified by means of affinity chromatography; and
Figure 9 illustrates the specificity of interaction between TNFα and 8 Δ TNF (144-157) immobilized on plastic microcontainers;
Figure 10 represents the kinetic curves of degradation of pro-endothelin and pro-endothelin 16-32 by the action of enzyme alpha-chymotrypsin;
Figure 11 represents the binding of the biotinized derivative of ET (16-32) with plates sensitized with 8 Δ ET (16-29) in the presence and in the absence of alpha-chymotrypsin; and
Figure 12 illustrates the bond of the biotinylated derivative of ET (16-32) with plates sensitized with 8 Δ ET (16-29) previously treated with alpha-chymotrypsin both in the presence and in the absence of inhibitors.

This invention has been employed for synthesizing sequences of nonlinear peptides which are hydropathically complementary to the site 16-29 of the Big Endothelin (Yanagisawa et al., Nature 322, 411 (1988)) [1-38], and to the site 144-157 of the TNFα (Bentler and Cerami, Ann. Rev. Immunol., 7:625, 1989), so obtaining a better exploitation of the same for the immobilization on solid phases. The peptides can be synthesized on the basis of procedures which are already known to those who are skilled in the art, in solution or through solid-phase synthesis, starting from a polylysine nucleus formed by the covalent union of seven lysine residues as illustrated in Figure 1A. The peptide 8 Δ ET [16-29], whose structure is shown in Figure 1B, has been synthesized by solid-phase synthesis following a Fmoc procedure (Dryland and Sheppard, TH 44, 859 (1988)) employing as the solvent system N-methylpyrrolidone/di-chloromethane, employing an automatic synthesizer Applied Biosystem 431A, and following the hints of the maker. Similarly, the nonlinear peptide 8 Δ TNF [144-157] has been synthesized, whose sequence is shown in Figure 7B. The synthesis and purification procedures for the peptides are largely represented and illustrated in the literature, and such procedures are well known to those who are skilled in the art. The peptide 8 Δ ET [16-29] has been employed for preparing an affinity column for the purification of Big ET from mixtures containing 20 different peptides, each one at a concentration of 1 mg/ml. As is shown in the following examples, affinity columns prepared with the 8 Δ ET [16-29] peptide show a higher binding capacity, i.e. they are capable of purifying higher amounts of Big ET with respect to columns prepared through the immobilization of the peptide Δ ET [16-29] (RKFLAGLRARRLKF) in equimolar amounts, said peptide representing the peptide unit which is repeated 8 times in 8 Δ ET [16-29]. The higher capacity obtained through the immobilization of 8 Δ ET [16-29] stems from the fact that just some of the eight peptide chains take part in the covalent interaction with the solid phase, whereas the other ones are free to interact non-covalently with the Big ET. On the contrary, in the case of the column prepared with the employment of Δ ET [16-29], the peptide is linked to the solid phase either through the N-terminal alpha-amino group or through the epsilon-amino groups of the lysines present in the positions 2 and 13 in the peptide sequence. In both cases, the peptide is not homogeneously immobilized, and anyway the closeness to the solid phase can cause remarkable decreases in the binding capacity for the Big ET. Such effects are quite common indeed also for other affinity systems and anyway they are largely illustrated in the literature. The usefulness of the present invention can be further appreciated, as is disclosed in the following examples, in the case of realizing analytical methodologies for the quantitative determination of said Big ET.

According to a particular embodiment of the present invention, the peptide 8 Δ ET [16-29] can be immobilized non-covalently on suitable plastic microcontainers which are then treated with proper protein solutions in order to remove the presence of any possible non-specific interaction sites, and then they are washed employing suitable solutions. Big Endothelin [1-38] or [16-29], labelled with suitable reactants, can then be added to each microcontainer, the whole being then kept under incubation in order to cause the interaction to occur. The container can then be washed in order to remove the excess Big Endothelin [1-38] or [16-29] that has not reacted, and then they are examined to determine the amount of Big ET or of its derivative [16-29] that has reacted. The technical literature largely disclosed

both the labelling methods, which can be realized through the introduction of radioisotopes, enzymes, fluorescent or chromophore compounds, or biotin, and the methods for the determination of such labelled compounds, and all said methods are well known to those who are skilled in the art. The strength of the non-specific interaction can be determined by incubating Big Endothelin [1-38], labelled in the presence of unlabelled excess Big ET, and analyzing the data obtained according to Scatchard's procedure. The specificity of interaction between the peptides which are the object of this invention can be determined through incubation within microcontainers derivatized with 8 Δ ET [16-29] with different peptides and proteins labelled in the presence or in the absence of unlabelled material. Further indications about specificity can be obtained through incubation of the fragments 16-21 or 22-32 of Big Endothelin [1-38] which have a very low affinity towards the peptide 8 Δ ET [16-29]. Similarly to the case of the peptide 8 Δ ET [16-29], the peptide 8 Δ TNF [144-157] has been immobilized both on a solid support for the preparation of affinity columns for the purification of TNFα, both on microplates for realizing a test to determine TNF quantitatively.

The usefulness of this invention is also evident, as illustrated in the examples 5, 7 and 8, in the case when the micromolecule of interest to be purified or to be analyzed contains more than just one site with the same sequence idropathically complementary to the immobilized multimeric ligand. In that case, the polyvalence of the multimeric ligand is characterized by an increase in the binding affinity for the molecule of interest. A general situation in the application of the present procedure can occur in the case of proteins made up of different sub-units which are all equal as regards the amino acid sequence and the structure. A peptide which is hydropathically complementary to an exposed site of such proteins can be synthesized according to the process which is the object of this invention in the octameric form, immobilized on a solid phase, and employed for purifying or for determining quantitatively the oligomeric protein of interest. The presence of a number of binding sites increases indeed the affinity exponentially, as in the case of bivalent antibodies which is largely disclosed in the literature. The observed increase of the binding affinity of the complementary multimeric peptides is applied clearly not only to the field of purifications carried out through the affinity chromatographic method or to the field of realization of analytical procedures. Indeed, as is evident to all those who are skilled in the art, the possibility of creating synthetic molecules which are able to become associated to protein macromolecules (proteins, receptors, antibodies, and so on)with high affinity (i.e. having low values of the dissociation constants) is largely exploited in the pharmacological field, for designing new synthetic drugs having activities closely directed towards a particular macromolecule having important biological function. A particular advantage in the use of such multimeric compounds with respect to the use of single peptides, is to be found both in the increased affinity, and in the higher stability with respect to the action of the proteolytic enzymes which are normally present in biological fluids. Indeed, the high molecular weight that results from the union of many peptide units gives the compound a higher steric inaccessibility as regards the active proteolysis site of the enzymes. Moreover, it is evident to those who are skilled in the art that it is possible to change, according to the various cases and the various specific applications, both the number of peptide units linked to the polylysine nucleus, and to introduce further residues in order to further space the peptide chains apart, without departing from the objects of the present invention.

EXAMPLE 1

Solid-phase synthesis of 8 Δ ET [16-29]

The sequence of the peptide hydropathically complementary to the sequence 16-29 of the human Big Endothelin [1-38] (HLDIIWVNTPEHIV) has been obtained on the basis of the method AMINOMAT previously disclosed by the same authors (Italian Patent Application No. 20755 A/90) and called Δ ET [16-29] (RKFLAGLRARRLKF). The peptide Δ ET [16-29] has been then synthesized by means of a solid-phase synthesis, employing a Fmoc methodology and exploiting standard coupling procedures of the single amino acid through the formation of active esters with dicyclohexylcarbodiimide/hydroxybenzotriazole [DCC/HOBt]. Similarly, the sequence 16-29 of the Big Endothelin has been synthesized. The peptides after the synthesis have been subjected to the removal of the protection groups and have been detached from the solid support according to standard procedures, and purified until obtaining chromatographic homogeneity by means of RP-HPLC. The octadentate polylysine nucleus employed for synthesizing the multiple peptide 8 Δ ET [16-29] (Figure 1A) has been obtained through successive coupling operation in three cycles of alpha-Fmoc-Lysine (epsilon-Fmoc) on the initial solid support (resin Fmoc-Glycine-HMP). The sequence Δ ET [16-29] has been then synthesized on the initial polylysine nucleus (Figure 1B) as disclosed previously. The multiple peptide, after removal of the protection groups and after detaching the same from the resin, has been purified from the low molecular weight contaminants by dialysis against 0.1 M acetic acid for two days. Finally the dialyzed material has been frozen and lyophilized. Following the same scheme, the peptides ET [16-29] and 8ET [16-29] have also been prepared, whose structure is given respectively in Figures 1C and D.

EXAMPLE 2

Preparation od solid phase for affinity chromatography

The peptides Δ ET [16-29] and 8 Δ ET [16-29] have immobilized on a support previously activated through the introduction of epoxide groups (EUPERGIT C30 N), employing the same peptide/support ratio. The peptides 8 Δ ET [16-29] (2 mg) and Δ ET [16-29] (2 mg) have been dissolved separately in 10 ml of 0.1 M $NaH_2CO_3$, 0.5 M NaCl, pH 8.5, and added separately to two aliquots of 1 g of EUPERGIT C30 N. The mixtures have been incubated for 24 hours under stirring at room temperature. The introduction of the peptides within the resin has been followed by means of RP-HPLC, and after 24 hours an amount of about 80 % of the peptide initially present in the two preparations turned out to be covalently linked to the support. Such incorporation percentage has been then confirmed by analysis of amino acids, which analysis were carried out on weighed amounts of the two derivatized resins.

The two derivatized peptide supports were employed for filling two glass columns for affinity chromatography (80 x 6.6 mm I.D., total volume 2.3 ml) that were equilibrated with 0.1 M TRIS* HCL, at pH 6.8 and with a flowrate of 1 ml/min. The eluent was continuously monitored by means of optical absorption determinations at 280 nm wavelength. For determining the capacity of the columns, 500 microlitres of ET [16-29] dissolved at a concentration of 1 mg/ml in the elution buffer were injected. Under the same elution conditions, the column prepared with the peptide 8 Δ ET [16-29] (Figure 2A) retains the injected material fully, whereas the column prepared with Δ ET [16-29] retains just 50% of the same (Figure 2B).

EXAMPLE 3

Preparation and purification of of Biotin ET [1-32]

The labelling of ET [1-32] for realizing analytical tests on plastic microcontainers was carried out through derivatization of the peptide with biotin according to the following procedure:

1) 2.5 mg of ET [1-32] was dissolved in 1 ml of 20 mM NaAc, at pH 6.0, and 400 microl. of a solution at a concentration of 2 mg/ml of biotin aminocaproate-N-hydroxysuccinimide ester in ethanol/water 1/1 was added. The mixture was then incubated under stirring for 5 hours at room temperature.
2) The reaction mixture was then diluted 1/1 with 50 mM TRIS, at pH 6.8, and 1 ml was then injected into the affinity column prepared with the peptide 8 Δ ET [16-29] and put in equilibrium with 50 mM TRIS at pH 6.8, at a flowrate of 1 ml/min. The effluent was then monitored by recording the optical absorption at 280 nm. After complete elution of the material that had not been kept and was made up of the excess reagent as well as of reaction by-products, the eluent was changed to 0.1 M HAc, pH 3.0, as shown in Figure 3B. The purified product, called Biotin ET [1-32] was then freeze-dried and employed directly for the successive objects.

EXAMPLE 4

Interaction of biotin-ET [1-32] with 8 Δ ET [16-29]-sensitized microplates

Some 96-well polystyrene plates (NUNC) have been sensitized with 100 microl/well of 8 Δ ET [16-29] solutions in a 0.1 M sodium carbonate buffer at pH 9.6, at various concentrations (2.0, 0.5, 0.2, 0.1, 0.0 microg/ml) for one night at 4°C. The plates were then washed three times with a 0.15 M sodium chloride, 0.5 M sodium phosphate solution at pH 7.3 (PBS) through repeated filling and emptying operations. At the end of such operations, each well was filled with 200 microl of a bovine serum albumin (BSA) solution containing 3 % PBS, and then incubated for 2 hours at room temperature. The plates were then washed again for three times with PBS, and each well was filled with 100 microl of a Biotin-ET [1-32] solution at various concentrations (0.2, 0.1, 0.05, 0.0 microg/ml) in PBS diluted at the ratio of 1/1 with distilled water containing 0.5 % BSA (PBS-B) this operation being followed by incubation for 2 hours at room temperature. At the end of the operations the plates were washed again with PBS (8 times) and filled with 100 microl/well of a streptavidin-peroxidase solution (Sigma Chemical Company), diluted in the ratio of 1/1,000 with PBS-B containing 0.05 % Tween 20. After one-hour incubation at 37°C, the plates were washed with PBS 8 times and filled with 100 microl/well of a 1 mg/ml o-phenilendiamine solution in 0.1 M sodium citrate buffer at pH 5 containing 5 mM hydrogen peroxide. The chromogenic reaction was carried out for 30 minutes at 37°C and stopped through the addition of .25 microl. of 4.5 M sulfuric acid. The optical density of each well was then determined by means of a microplate reader (Model 3550 Microplate Reader, Biorad). The results obtained which are shown in Figure 3, pointed out the effective binding between the Biotin-ET [1-32] and the 8 Δ ET [16-289]-sensitized wells. The binding, as expected, turned out to be dependent on both the Biotin-ET [1-32] and the 8 Δ ET [16-29] concentrations. More particularly, the employment

of 8 Δ ET [16-29] at the concentration of 0.5 mg/ml in the step of sensitization of the plates and Biotin-BT [1-32] at the concentration of 0.2 microg/ml seems to be sufficient to generate a measurable signal for developing a competitive test with ET [1-32].

EXAMPLE 5

Test of competitive binding for ET [1-32]

In order to evaluate the specificity of the bond between Biotin-ET [1-32] and the 8 Δ ET [16-29]-sensitized wells, as disclosed in example 4, a binding competition test of Biotin-ET [1-32] with ET [1-32] and with two negative-control peptides C1 and C2 was carried out, the binding occuring with 8 Δ ET [16-29]-coated wells. The experiment was carried out following the procedure disclosed in example 4 with fixed concentrations of 8 Δ ET [16-29] (0.5 microg/ml) and of Biotin-ET [1-32] (0.2 microg/ml) and in the presence of various concentrations of competitor peptides between 1 and 1,000 microg/ml.

The results obtained which are shown in Figure 5, point out the effective competition of the binding between Biotin-BT [1-32] and 8 Δ ET [16-29] with ET [1-32] at concentrations between 5 and 250 microg/ml but not with C1 and C2. This suggests that the observed interaction between the complementary peptides is specific. The specificity of the interaction was further confirmed through competition experiments with ET [16-21]. In that case no appreciable binding competition was observed at the concentrations employed in the tests. This can be easily interpretated in terms of drastic reduction in the affinity due to the lack of 11 residues in the hydropathically complementary region which are necessary for stabilizing the interaction. It was observed, in agreement with such hypothesis, that the peptide ET [16-29], containing the whole complementary region is on the contrary capable of competition. As a conclusion, the binding between Biotin-ET [1-32] and 8 Δ ET [16-29] observed on the polystyrene plates is specific and it depends on the hydropathically complementary regions of the two peptides, and is exploitable for the determination of molecules containing said regions, as for instance Big-Endothelin and pro-Endothelin.

EXAMPLE 6

Determination of the relative affinity of ET [16-29] and 8ET [16-32] towards 8 Δ ET [16-29]

The column containing the peptide 8 Δ ET [16-29], prepared as disclosed in example 2, was put in equilibrium with 1M $NH_4Ac$ at pH 5,7, at a flowrate of 1.0 ml/min. Under such conditions of high ionic strength, the interactions between complementary peptides turn out to be remarkably reduced as disclosed in the literature (Fassina et al., J. Biol. Chem. 264: 11252, 1989) and it is possible to observe just a delay in the elution volume of the interacting compound with respect to the dead volume of the column instead of a full adsorption. It is possible to calculate from the elution volume the interaction dissociation constant Km/p on the basis of the equation:

$$\frac{1}{V-V_o} = \frac{K_{M/P}}{M_T} + \frac{[P]}{M_T}$$

wherein V is the elution volume, $V_o$ is tie dead volume of the column, $M_t$, is the column capacity and P is the amount of the eluted component (Fassina and Chaiken, Adv. in Chromatogr., 27:247, 1987). In such conditions, the peptide ET [16-29] undergoes an elution delay equal to 3 ml (Figure 6A) whereas the peptide 8ET [16-29] is not eluted even after 100 minutes, and it is necessary to change the eluent to 0.1 M HAc for the complete elution of the same (Figure 6B). The dissociation constant of the peptide ET [16-29] thus turns out to be at least 50 times as high with respect to that obtained with the peptide 8 Δ ET [16-29]. This means that the polyvalence in the interaction between 8 Δ ET [16-29] and 8ET [16-29] increases the association extent by about 2 orders of magnitude.

EXAMPLE 7

Design and solid-phase synthesis of 8 Δ TNF [144-157]

The sequence of the peptide hydropathically complementary to the sequence 144-157 of the Tumor Necrosis Factor (TNFalpha) (FAESGQVYFCIIAL ) has been obtained on the basis of the AMINOMAT procedure which has been disclosed previously (Italian Patent Applications No. 21396 A/89 and No. 20755 A/90) and called Δ TNF [144-157] [DYLAGFKAHGKKYR] The peptide 8 Δ TNF [144-157] was then synthesized by solid-phase synthesis with Fmoc methodology starting from an octadentate polylysine nucleus provided with glycine and arginine spacers as illustrated

in Figure 7A. Similarly to what has been disclosed previously, the peptide 8 Δ TNF [144-157] (Figure 7B) was then deprotected, detached from the resin and purified through dialysis against 0.1 M acetic acid for two days. Finally the dialyzate was frozen and freeze-dried. The peptide 8 Δ TNF [144-157] (5 mg) was then immobilized on a previously activated support (1 g) with epoxide groups (EUPERGIT C30 N), exploiting the same conditions as those reported previously. The derivatized peptide support was then employed for filling a glass column for affinity chromatography (80 x 6.6 mm I.D., total volume 2.3 ml), which was then equilibrated with 0.1 M TRIS HCl at pH 6.8 at a flowrate of 2.0 ml/min. In order to determin the recognition properties of the column towards TNF alpha, injections were made of 100 microl of TNF alpha at a concentration of 0.5 mg/ml dissolved in the elution buffer. After about 10 minutes the buffer was changed to 0.1 M acetic acid in order to allow the elution of the adsorbed material to occur (Figure 8A). The material eluted with acetic acid was then analyzed by RP-HPLC to confirm the occurrence of the recognition between TNF alpha and 8 Δ TNF [144-157] (Fig. 8B).

EXAMPLE 8

Interaction between Biotin TNF and 8 Δ TNF [144-157]-sensitized microplates

50 microl of a solution of 8 Δ TNF [144-157] at a concentration of 0.04 mg/ml were added to each well of a 96-well, PVC plates, in a 0.1 M sodium bicarbonate buffer at pH 9.6. The plates were then incubated for 1 hour at room temperature and then washed three times with PBS. Each well was then filled with 200 microl of 3 % bovine serum albumin (DSA) solution in 0.025 M sodium phosphate buffer at pH 6.5, containing 0.075 M NaCl (PBS). After one-hour incubation at room temperature, the plates were washed with PBS and filled with TNF/TNF-Biotin mixtures at various delutions in 1 % w/v PBS-BSA, containing 0.05 % v/v Tween 20, 10 KIU/ml Aprotinine, PMSF 10 microM, EDTA 10 microM, (PBS-TAPE) in the presence and in the absence of 1 % normal goat serum. The plates were then incubated again for 1 hour at room temperature and then washed with PBS. Each well was then filled with 100 microl of a streptavidin-peroxidase solution (Sigma) diluted 1/2,000 with PBS-TAPE and incubated for 1 hour at room temperature. Finally the plates were washed, filled with a chromogen ABTS (KPL) solution (100 microl/well) and incubated for 30 minutes at room temperature. The absorbance at 405 nm of each well was then determined by means of a microplate reader (BioRad Model 25550 EIA Reader). As shown in Figure 8, the binding between 8 Δ TNF [144-157] immobilized on the wells and TNF alpha-Biotin turns out to be specific and it can be in competition with TNF alpha both in the presence and in the absence of goat normal serum.

EXAMPLE 9

Determination of the proteolysis kinetics of the substrate ET (16-32) and pro-endothelin by means of alpha-chymotrypsin

The peptide pro-endothelin 1-38 was obtained from commercial sources (Novabiochem, CH). Solutions of such peptide and of the peptide ET 16-32 at a concentration of 0.1 mg/ml were prepared in 50 mM TRIS at pH 6.8, to which the addition was carried out of alpha-chymotrypsin aliquots, so obtaining an enzyme/substrate ratio of 1/1,000. The proteolysis reaction was followed in time by sampling after different incubation times at 20°C, aliquots of 50 microl for chromatographic analysis by means of RP-HPLC, employing a reverse -phase column ABI RP-300 30 x 2.1 mm I. D., equilibrated with $H_2O/CH_3CN/TFA$ 97/3/C.1 at a flowrate of 0.5 ml/min, and employing a linear elution gradient of the type:

| Time | $CH_3CN$ |
|---|---|
| 0 | 3 |
| 5 | 3 |
| 25 | 35 |
| 30 | 80 |
| 35 | 80 |
| 37 | 3 |

and monitoring the eluent by 225 nm wavelength. Under such conditions, the elution tines of the peptices and of the relative fragments were:

| Peptide | $T_{elution}$ (min) |
|---|---|
| ET (16-32) | 23 |
| ET (1-38) | 32 |
| ET (1-21) | 22 |
| ET (22-38) | 15 |
| ET (16-21) | 21.5 |
| ET (22-32) | 12 |

The degradation percentages were then calculated according to the formula:

$$\% \text{ degradation} = \frac{A_{T=0} - A_{T=x}}{A_{T=0}} \times 100$$

wherein $A_{T=0}$ corresponds to the area under the RP-HPLC peak relative to ET (16-22) or ET (1-38) in the absence of chymotrypsin, whereas $A_{T=x}$ corresponds to the area regarding the fragments ET (16-21) or ET (1-21) determined after a time x of treatment with alpha-chymotrypsin at 20°C. The degradation kinetics data of the two peptides ET (16-32) and pro-ET (1-36) are shown in Figure 10. The close correspondence of the two kinetic curves obtained for the degradation of the two peptides allows to set forth that the fragment 16-32 of pro-endothelin is an optimal alternative substrate for stucying the action of pro-endothelin specific proteolytic enzymes.

EXAMPLE 10

Test for detecting pro-endothelin specific proteolytic activities present in biological fluids

A) Preparation and purification of Biotin ET (16-32). The labelling of ET (16-32) for realizing analytical tests on plastic microcontainers was carried out derivatizing the peptide with biotin according to the following procedure:

1) 2.5 mg of ET (16-32) was dissolved in 1 ml of 20 mM NaAc, at pH 6.0, and 400 microl of a solution of biotin aminocaproate-N-hydroxysuccinimide ester at a concentration of 2 mg/ml in ethanol/water 1/1 was added. The mixture was then incubated under stirring for 5 hours at room temperature.
2) The reaction mixture was then diluted 1/1 with 50 mM TRIS at pH 6.8, and 1 ml was then injected into an affinity column prepared with the peptide 8 Δ ET (16-29). The purified product, which is called Biotin-ET (16-32) was then freeze-dried and employed directly for the next steps.

B) Preparation of sensitized plates

Some 96-well polystyrene plates (NUNC) were sensitized with 100 microl/well of a solutioon at a concentration of 200 microg/ml of 8 Δ ET (16-29) in 0.1 M sodium carbonate buffer at 9.6 for one hour at 20°C. The plates were then washed three times with a 0.15 M sodium chloride solution, 0.5 % sodium phosphate at pH 6.5 (PBS), through repeated filling and emptying operations. At the end of the operations, each well was filled with 200 microl of a 3 % bovine serum albumin (BSA) solution in PBS containing 0.1 mM PMSF, 0.1 mM EDTA, aprotinin 50 microg/ml and incubated for one hour at room temperature. The plates were then washed again 8 times with PBS.

C) Standard treatment

Each well was filled with 100 microl of a Biotin-ET (16-32) solution at various concentrations (10; 1; 0.1; 0.001 and 0.0 microg/ml) in PBS diluted in the ratio of 1/1 with distilled water containing 0.5 % BSA (PBS-B) and previously treated with variable concentration of alpha-chymotrypsin (10; 5; 1; 0.1; 0.0 microg/ml) and incubated for 1 hour at room temperature.

D) Development of plates

At the end of the operations, the plates were filled with 100 microl per well of a streptavidin-peroxidase solution (Sigma Chemical Company) diluted in the ratio of 1/1,000 with PBS-B containing Tween 20 at 0.05 % concentration. After one hour of incubation at room temperature, the plates were washed with PBS (8 times) and filled with 100 microl/well of 1 mg/ml o-phenylendiamine solution in 0.1 M sodium citrate buffer at pH 5, containing 6 mM hydrogen peroxide. The chromogenic reaction was carried out for 30 minutes at 20°C and then stopped by the addition of

100 microl of 0.1 M hydrochloric acid. The optical density of each well was then measured by means of a microplate reader (BioRad Model 3550 Microplate Reader). The results so obtained shown in Figure 11 pointed out that Biotin-ET (16-32) binds to the sensitized wells in a way proportional to its concentration, whereas if it is previously treated with chymotrypsin, it looses such binding capability. Hence it is immediately clear that the presence of enzymes capable of proteolysing ET (16-32) is easily detectable by incubating the sample under test with a standard Biotin-ET (16-32) solution and measuring as disclosed previously the amount of Biotin-ET (16-32) linked to the sensitized wells.

EXAMPLE 11

Test for detecting inhibitors of pro-endothelin specific proteolytic activities in biological fluids

A) Preparation of sensitized plates

Some 96-well plates were sensitized with the 8 Δ ET (16-29) peptide as disclosed previously in Example 10.

B) Standard treatment

Samples containing inhibitors of pro-endothelin specific proteolytic activities (100 microl) were added to 50 microl of a solution at a concentration of 1 microg/ml of alpha-chymotrypsin dissolved in 0.5 % BSA PBS at pH 6.8 and next 50 microl of a solution of biotin-ET (16-32) at the concentration of 1 microg/ml was added. 100 microl of such mixture was then added to the sensitized wells after 1 hour incubation period.

C) Development of plates

The development was carried out as disclosed previously in Example 10.

Data obtained and shown in Figure 12 point out clearly that the presence of inhibitors in the analyzed sample of pro-endothelin specific enzymes is easily evidenced by the positive reading values of the wells. When the degradation of biotin-ET (16-32) is inhibited, the amount of the biotinized substrate capable of binding to the wells is higher than that of samples that do not have such inhibiting activity.

## Claims

1. A process for the identification of a proteolytic activity (E) and/or of an inhibitor (I) of such activity (E), in a biological material, said process comprising:

   d) incubating a substrate A, comprising at least a labelled protein fragment having at least a proteolytic attack site for said proteolytic activity (E) with said biological material;
   e) allowing the reaction to convert the substrate (A) into fragments (C) and (D);
   f) measuring the amount of substrate A by means of a ligand (B) which is capable of recognizing said substrate (A) but not said fragments (C) and (D).

2. A process for the identification of proteolytic activities and/or of inhibitors of such activities according to claim 1 wherein said proteolytic activities and/or activities that inhibit the same are present in biological fluids or in fermentation broths or cell extracts.

3. A process for the identification of proteolytic activities and/or of activities capable of inhibiting the same according to anyone of the preceding claims wherein said protein fragment is labelled by means of radioisotopes, or of enzymes, or of fluorescent compounds or of chromophores or of biotin.

4. A process for the identification of proteolytic activities and/or inhibitors of such activities according to claim 3 , wherein said protein fragment is labelled by means of biotin derivatization and is evidenced by incubation with streptavidin conjugated with peroxidase.

5. A process for the identification of proteolytic activities and/or inhibitors of such activities according to anyone of the preceding claims , said process comprising also means for the separation of the complex protein-ligand fragment from the protein fragments which are not bound and from the proteolyzed derivatives.

6. A process for the identification of proteolytic activities and/or inhibitors of such activities according to claim 5 wherein said separation means are realized by immobilizing in the solid phase said ligand and washing said unbound protein fragments and said proteolyzed derivatives away.

**7.** A process for the identification of proteolytic activities and/or inhibitors of such activities according to claim 6 wherein said solid phase comprises plastic plates divided into compartments.

**8.** A process for the identification of proteolytic activities and/or inhibitors of such activities according to anyone of the preceding claims 5-7 comprising the step of determining said labelled protein fragments which are bonded to said ligand immobilized on a solid phase.

**9.** A process for the identification of proteolytic activities and/or inhibitors of such activities according to previous claims wherein said substrate comprises a fragment of pro-endothelin and said ligand comprises amino acid sequences which are hydropathically complementary to said fragment of pro-endothelin.

**10.** A process for the identification of proteolytic activities and/or inhibitors of such activities according to claim 9 wherein said fragment of pro-endothelin is made up of the sequence from the amino acid 16 to the amino acid 32, having the formula

HLDIIWVNTPEHIVPYG

and said ligand is the molecule 8 Δ ET (16-29).

**11.** A process for the identification of proteolytic activities and/or inhibitors of such activities according to claim 10 wherein said proteolytic attack site is between the amino acids 21 and 22 of said fragment 16-32 of pro-endothelin, such as to generate in the presence of proteolytic activities two fragments ET (16-21) and ET (22-32) which are uncapable of binding to said molecule 8 Δ ET (16-29).

## Patentansprüche

**1.** Verfahren zur Feststellung einer proteolytischen Wirkung (E) und/oder eines Inhibitors (I) einer solchen Wirkung (E) in einem biologischen Stoff, mit folgenden Verfahrensschritten:

d) Inkubation eines Substraktes A, welches mindestens ein etikettiertes Proteinfragment mit mindestens einem proteolytischem Angriffssitus für diese proteolytische Wirkung (E) mit diesem biologischen Stoff enthält;
e) die Reaktion vorsichgehen lassen bis sie das Substrakt (A) in Fragmente (C) und (D) umformt;
f) Messung der Menge von Substrakt (A) mittels eines Ligands (B), das das vorgenannte Substrakt (A) aber nicht die vorgenannten Fragmente (C) und (D) zu erkennen vermag.

**2.** Verfahren zur Feststellung von proteolytischen Wirkungen und/oder Inhibitoren solcher Wirkungen nach Anspruch 1, worin solche proteolytische und/oder inhibierende Wirkungen in biologischen Flüssigkeiten oder in Fermentationsbrühen vorhanden sind.

**3.** Verfahren zur Feststellung von proteolytischen Wirkungen und/oder Inhibitoren solcher Wirkungen nach je einem der vorhergehenden Ansprüche, worin das vorgenannte Proteinfragment durch Radioisotope oder Enzyme oder fluoreszierende Verbindungen etikettiert ist.

**4.** Verfahren zur-Feststellung von proteolytischen Wirkungen und/oder Inhibitoren solcher Wirkungen nach Anspruch 3, worin das vorgenannte Proteinfragment durch Biotinableitung etikettiert und durch Inkubation mit Streptavidin, die mit Peroxidase konjugiert ist, hervorgehoben wird.

**5.** Verfahren zur-Feststellung von proteolytischen Wirkungen und/oder Inhibitoren solcher Wirkungen nach je einem der vorhergehenden Ansprüche, worin auch Mittel vorgesehen sind, die zum Abscheiden der Legand_Proteinfragment-Komplexverbindung vom nicht gebundenen Proteinfragment und von den proteolizierten Derivaten dienen.

**6.** Verfahren zur Feststellung von proteolytischen Wirkungen und/oder Inhibitoren solcher Wirkungen nach Anspruch

5, worin die vorgenannten Abscheidemittel durch Immobilisierung in der festen Phase des vorgenannten Ligands und Abwaschen des ungebundenen Proteinfragments und der proteolizierten Derivaten erhalten werden.

**7.** Verfahren zur Feststellung von proteolytischen Wirkungen und/oder Inhibitoren solcher Wirkungen nach Anspruch 6, worin die vorgenennte feste Phase Kunsstoffplatten, die in Abteilungen unterteilt sind, enthält.

**8.** Verfahren zur Feststellung, von proteolytischen Wirkungen und/oder Inhibitoren solcher Wirkungen nach je einem der vorhergehenden Ansprüche, worin ein Verfahrenschritt zur Feststellung der etikettierten Proteinfragmente, die an das vorgenannte in der festen Phase immobilisierte Ligand gebunden sond, vorgesehen ist.

**9.** Verfahren zur Feststellung von proteolytischen Wirkungen und/oder Inhibitoren solcher Wirkungen nach den vorbergehenden Ansprüchen, worin das vorgenannte Substrakt ein Proendothelinfragment enthält und das vorgenannte Ligand Aminosäuresequenzen enthält, die zum vorgenannten Proendothelinfragment hydropathikalisch komplementär sind.

**10.** Verfahren zur Feststellung von proteolytischen Wirkungen und/oder Inhibitoren solcher Wirkungen nach Anspruch 9, worin das vorgenannte Proendothelinfragment aus der Frequenz von Aminosäuren von 16 bis 32 mit der Formel

HLDIIWVNTPEHIVPYG

besteht und das vorgenannte Ligand ist das Molekül 8 Δ ET (16-29).

**11.** Verfahren zur Feststellung von proteolytischen Wirkungen und/oder Inhibitoren solcher Wirkungen nach Anspruch 10, worin der vorgenannte proteolitische Eingriffsitus sich zwischen den Aminosäuren 21 und 22 befindet, um so in Anwesenheit von proteolytischen Wirkungen zwei Fragmente ET (16-21) und ET (22-32) zu erzeugen, die sich mit dem vorgenannten Molekül 8 Δ ET zu binden nicht imstande sind.

## Revendications

**1.** Procédé pour l'identification d'activités protéolytiques (E) et/ou d'un inhibiteur (I) de celles-ci dans une matière biologique, ledit procédé comprenant:

d) incubation d'un substract A, comprenant au moins un fragment de protéine étiqueté ayant au moins un site d'attaque prbtéolytique pour ladite activité protéolytique (E) avec ladite matière biologique;
e) laisser que la réaction convert le substract (A) en fragments (C) et (D);
f) meusure de la quantité du substrat (A) ou moyen d'un liant (B) qui est capable de reconnaître ledit substrat (A) mais non lesdits fragments (C) and (D).

**2.** Procédé pour l'identification d'activités protéolytiques et/ou des inhibiteur de telles activités selon la revendication 1, dans lequel lesdites activités protéolitiques et/ou les activités qui inhibitent celles-ci sont présentes dans fluids biologiques ou dans bouillons de fermentation ou extraits de cellules.

**3.** Procédé pour l'identification d'activités protéolytiques et/ou d'activités capables de inhibir celles-ci selon l'une quelconque des revendications précédentes, dans lequel ledit fragment de protéine est étiqueté au moyens de radioisotopes ou d'enzymes ou de composition fluorescentes ou de chromofores ou de biotine.

**4.** Procédé pour l'identification d'activités protéolytiques et/ou d'activités capables de inhibir celles-ci selon la revendication 3, dans lequel ledit fragment de protéine est étiqueté au moyen de la derivatisation de biotine et est mis en évidence par incubation avec streptavidine conjungée avec peroxydase.

**5.** Procédé pour l'identification d'activités protéolytiques et/ou d'activités capables de inhibir celles-ci selon l'une quelconque des revendications précédentes, dans lequel des moyens sont prévus pour la séparation du complex de fragment de protéine et de liant des fragments de protéine qui ne sont pas liés et des derivés proteolysés.

**6.** Procédé pour l'identification d'activités protéolytiques et/ou d'activités capables de inhibir celles-ci selon la revendication 5, dans lequel lesdits moyens de séparation sont réalisés par immobilisation dans la phase solide dedit

liant et par lavage desdit fragments de protéine non liés et desdits derivés protéolysés.

**7.** Procédé pour l'identification d'activités protéolytiques et/ou d'activités capables de inhibir celles-ci selon la revendication 6, dans lequel ladite phase solid comprend de plaques en plastique divisées en compartiments.

**8.** Procédé pour l'identification d'activités protéolytiques et/ou d'activités capables de inhibir celles-ci selon les revendications précédentes 5-7, comprenant la phase de détermination desdits fragments de protéine etiquetés qui sont liés audit liant immobilisé sur une phase solide.

**9.** Procédé pour l'identification d'activités protéolytiques et/ou d'activités capables de inhibir celles-ci selon les revendications précédentes, dans lequel ledit substrat comprend un fragment de pro-endotheline et ledit liant comprend des séquences de aminoacides, qui sont hydrophaticalement complementaires audit fragment de pro-endotheline.

**10.** Procédé pour l'identification d'activités protéolytiques et/ou d'activités capables de inhibir celles-ci selon la revendication 9, dans lequel ledit fragment de pro-endotheline est réalisé de la séquence de l'aminoacide 16 à l'aminoacide 32, ayant la formule

HLDIIWVNTPEHIVPYG

et ledit liant est la molécule 8 $\Delta$ ET (16-29).

**11.** Procédé pour l'identification d'activités protéolytiques et/ou d'activités capables de inhibir celles-ci selon la revendication 10, dans lequel ledit site d'attaque protéolitique se trouve entre les aminoacides 21 et 22 dudit fragment 16-32 de pro-endotheline, de façon à engendre en presence des activités protéolitiques deux fragments ET (16-21) et ET (22-32) qui sont incapables de se lier à ladite molécule 8 $\Delta$ ET (16-29).

FIG. 1A

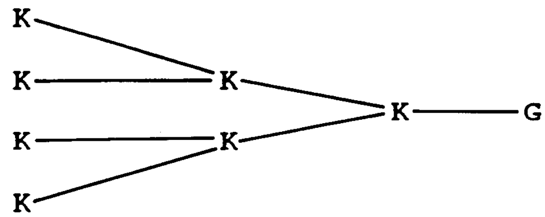


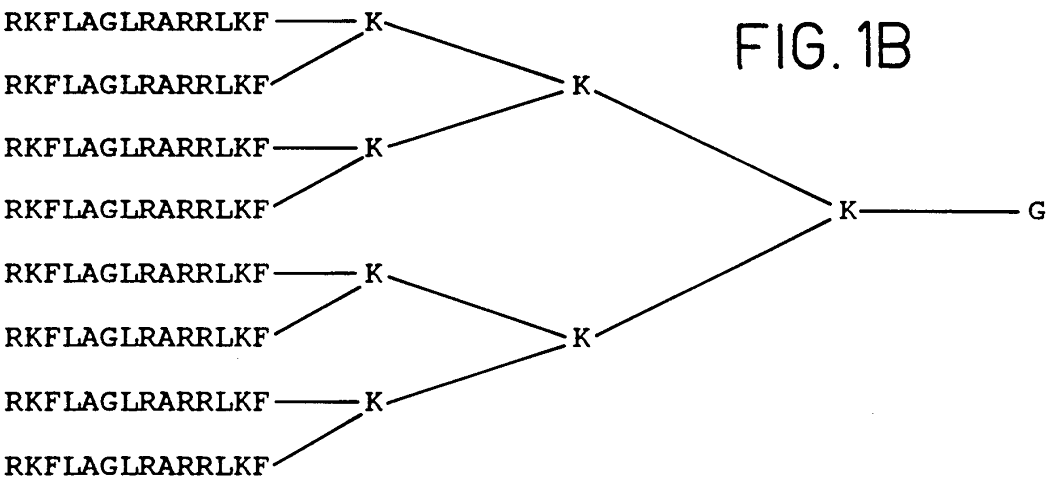


FIG. 1B

FIG. 1C HLDIIWVNTPEHIV

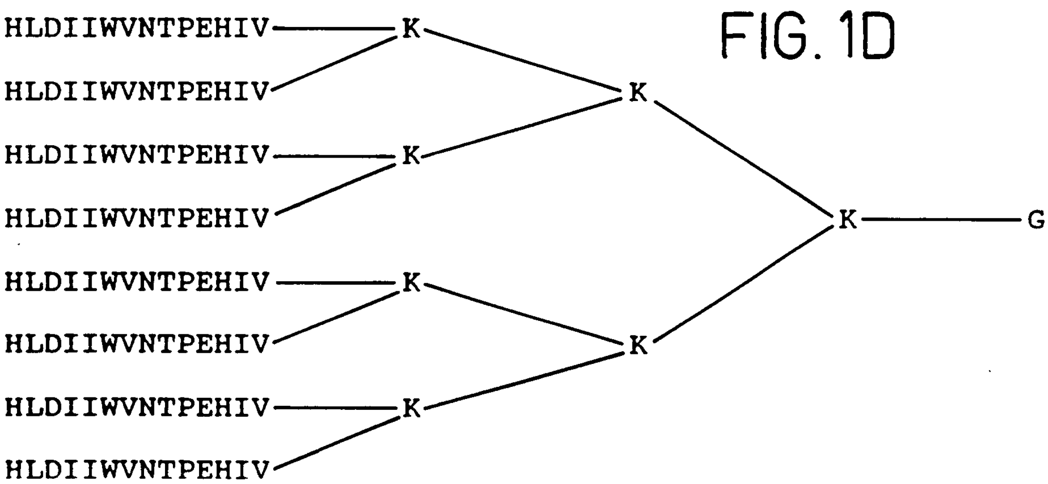


FIG. 1D

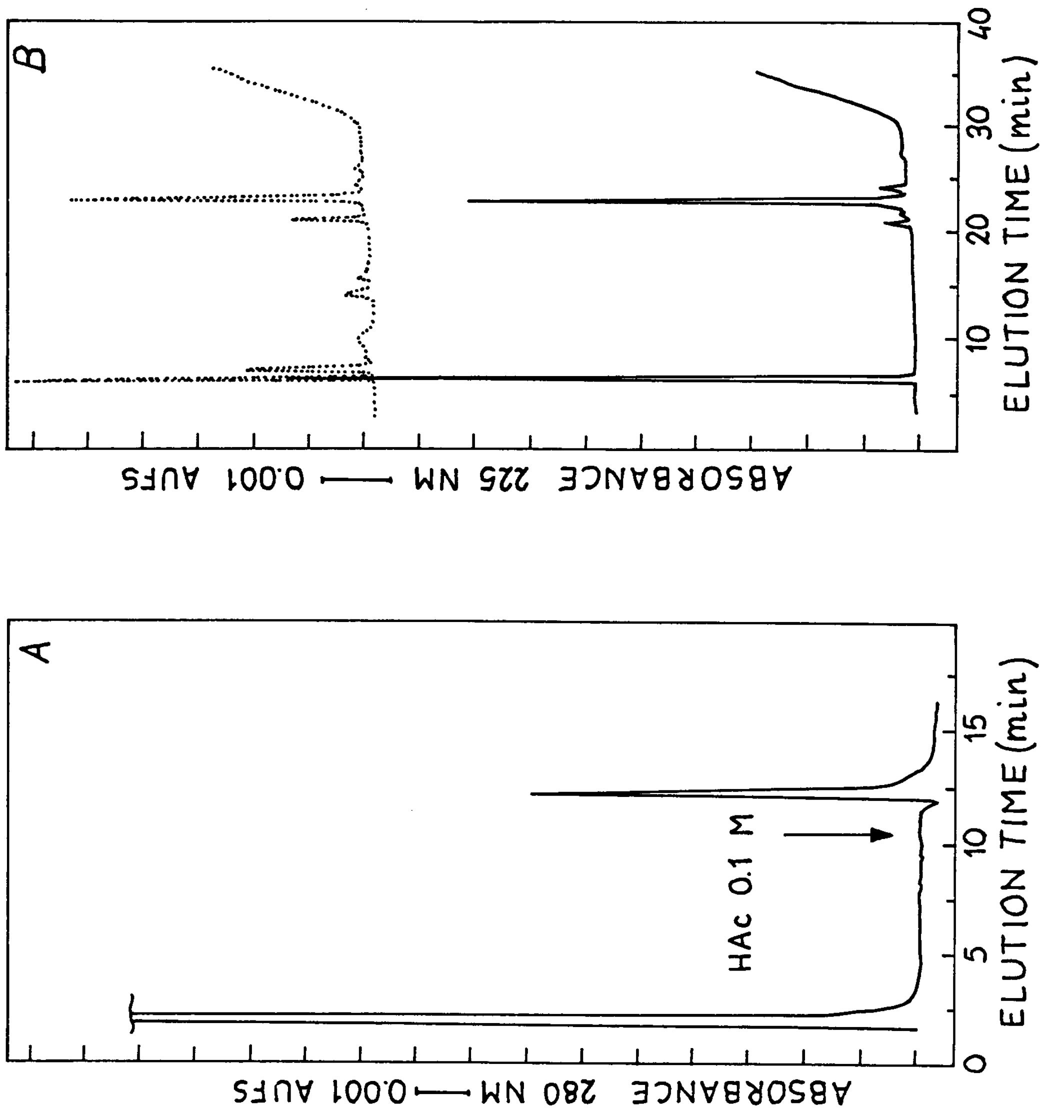
A
B
HAc 0.1 M
ABSORBANCE 280 NM —— 0.001 AUFS
ABSORBANCE 225 NM —— 0.001 AUFS
ELUTION TIME (min)
0
5
10
15
20
30
40

FIG. 3

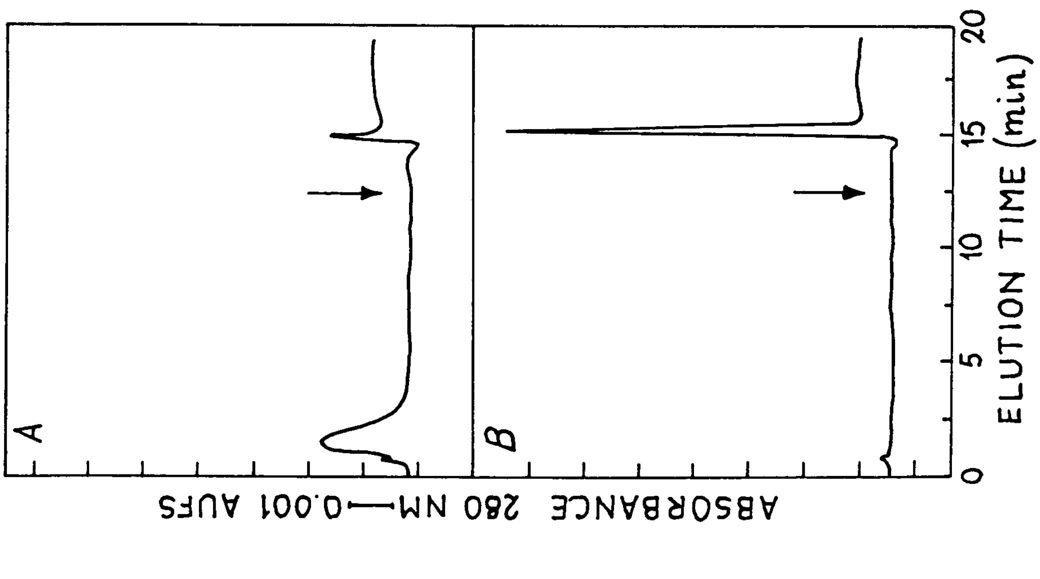
A
B
ABSORBANCE 280 NM —— 0.001 AUFS
ELUTION TIME (min)
0
5
10
15
20

FIG. 2

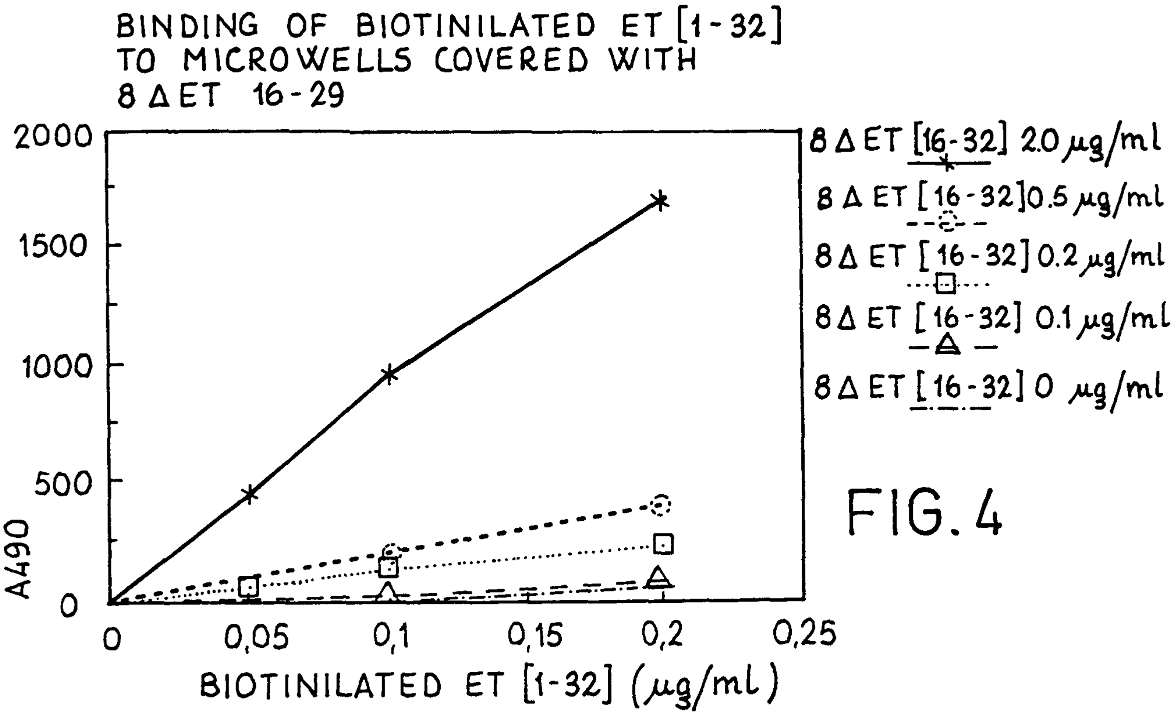
BINDING OF BIOTINILATED ET [1-32]
TO MICROWELLS COVERED WITH
8 Δ ET 16-29
2000
1500
1000
500
0
A490
0
0,05
0,1
0,15
0,2
0,25
BIOTINILATED ET [1-32] (μg/ml)
8 Δ ET [16-32] 2.0 μg/ml
8 Δ ET [16-32] 0.5 μg/ml
8 Δ ET [16-32] 0.2 μg/ml
8 Δ ET [16-32] 0.1 μg/ml
8 Δ ET [16-32] 0 μg/ml

FIG. 4

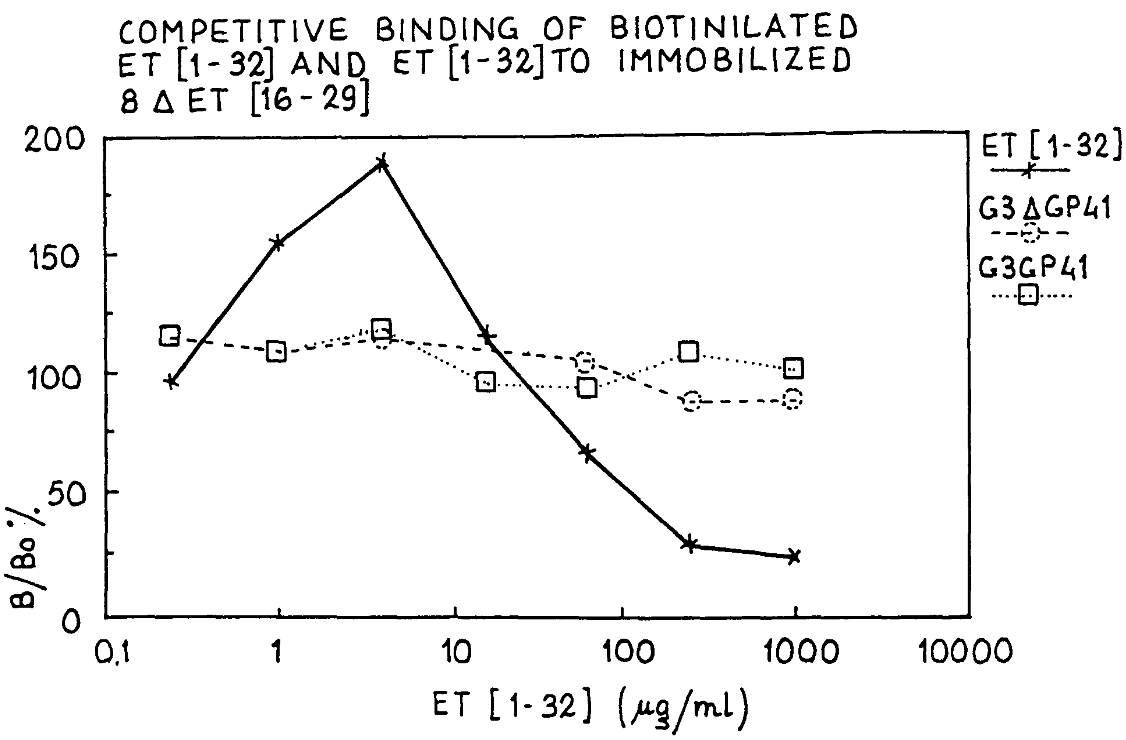
COMPETITIVE BINDING OF BIOTINILATED
ET [1-32] AND ET [1-32] TO IMMOBILIZED
8 Δ ET [16-29]
200
150
100
50
0
B/Bo %
0.1
1
10
100
1000
10000
ET [1-32] (μg/ml)
ET [1-32]
G3 Δ GP41
G3GP41

FIG. 5

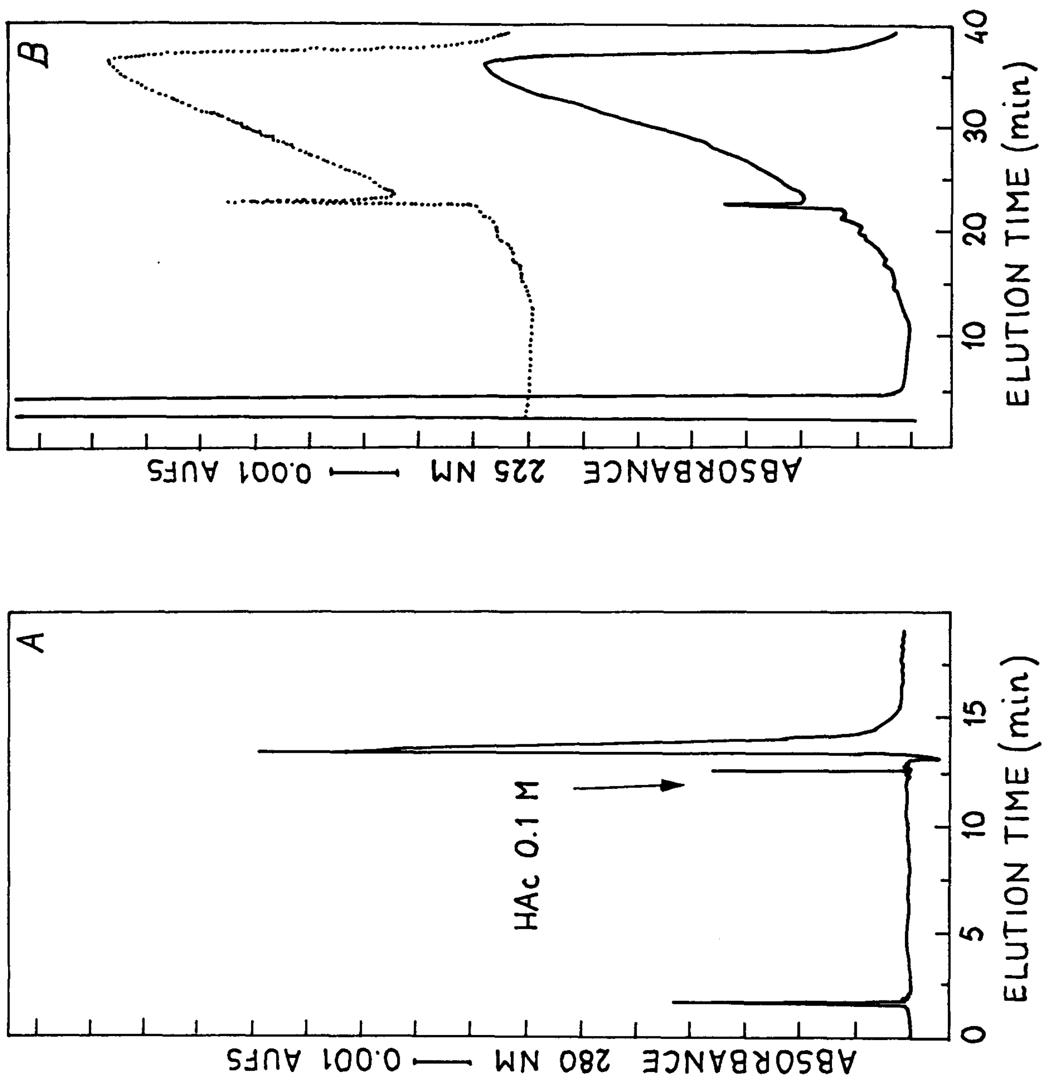
A
B
ABSORBANCE 225 NM ⟼ 0.001 AUFS
ABSORBANCE 280 NM ⟼ 0.001 AUFS
HAc 0.1 M
0
5
10
15
20
30
40
ELUTION TIME (min)

FIG. 8

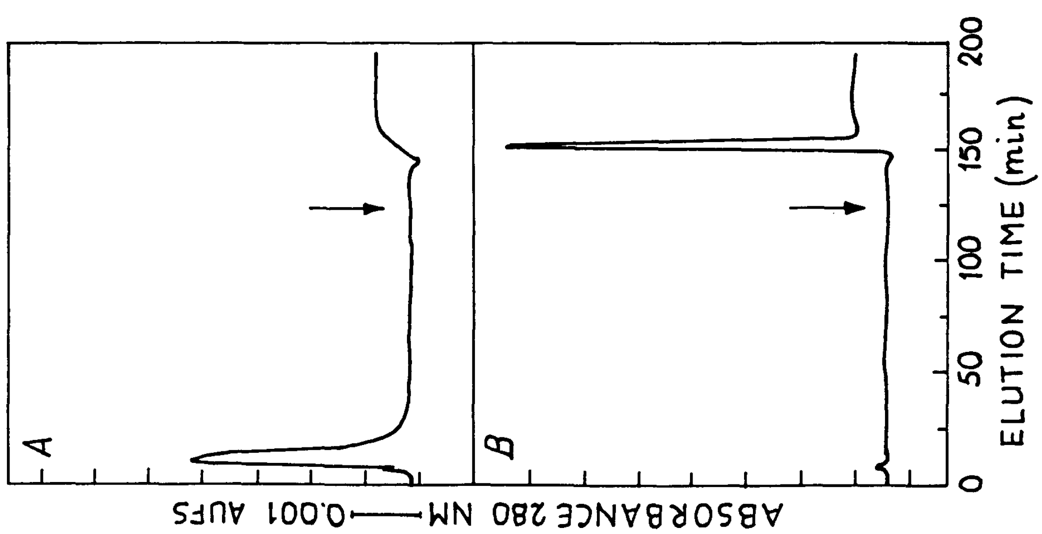
A
B
ABSORBANCE 280 NM ⟼ 0.001 AUFS
0
50
100
150
200
ELUTION TIME (min)

FIG. 6

FIG. 7a

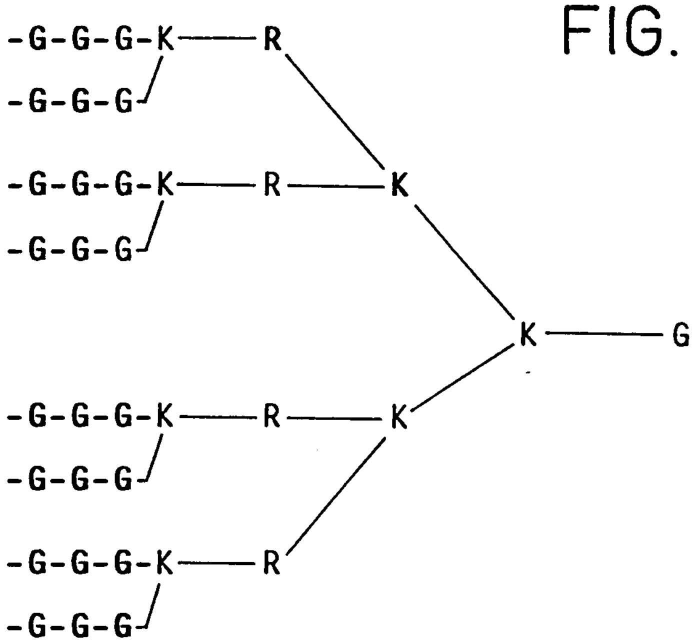
-G-G-G-K——R
-G-G-G
-G-G-G-K——R——K
-G-G-G
K——G
-G-G-G-K——R——K
-G-G-G
-G-G-G-K——R
-G-G-G

FIG. 7b

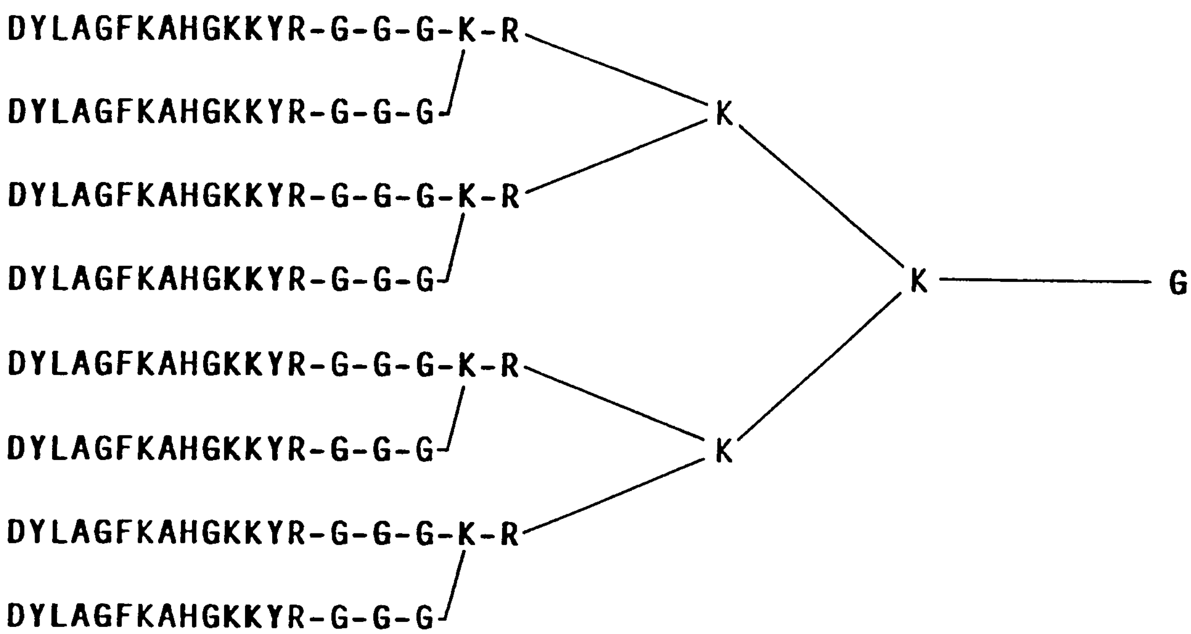
DYLAGFKAHGKKYR-G-G-G-K-R
DYLAGFKAHGKKYR-G-G-G
K
DYLAGFKAHGKKYR-G-G-G-K-R
DYLAGFKAHGKKYR-G-G-G
K——G
DYLAGFKAHGKKYR-G-G-G-K-R
DYLAGFKAHGKKYR-G-G-G
K
DYLAGFKAHGKKYR-G-G-G-K-R
DYLAGFKAHGKKYR-G-G-G

FIG. 9

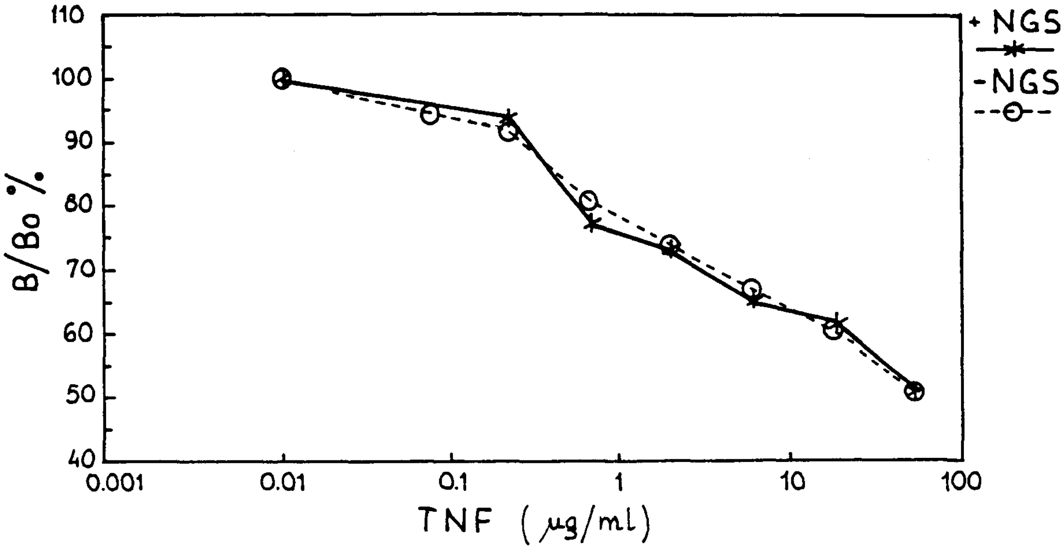


PROTEOLYTIC DEGRADATION KINETICS

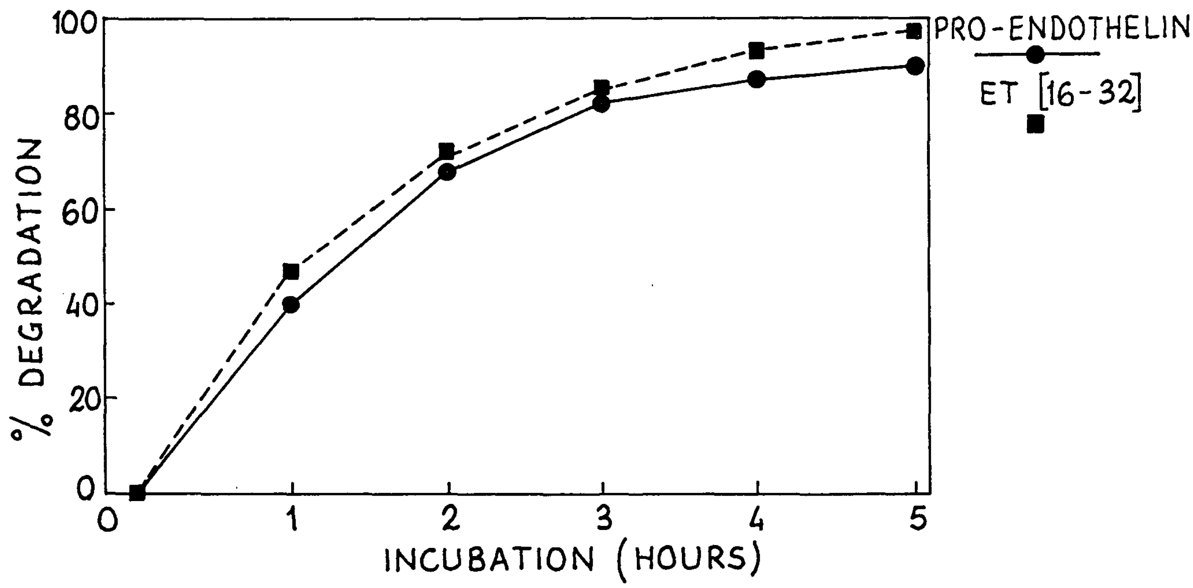


PRO-ET [50µg/ml]
ET[16-32] [50µg/ml]

FIG. 10

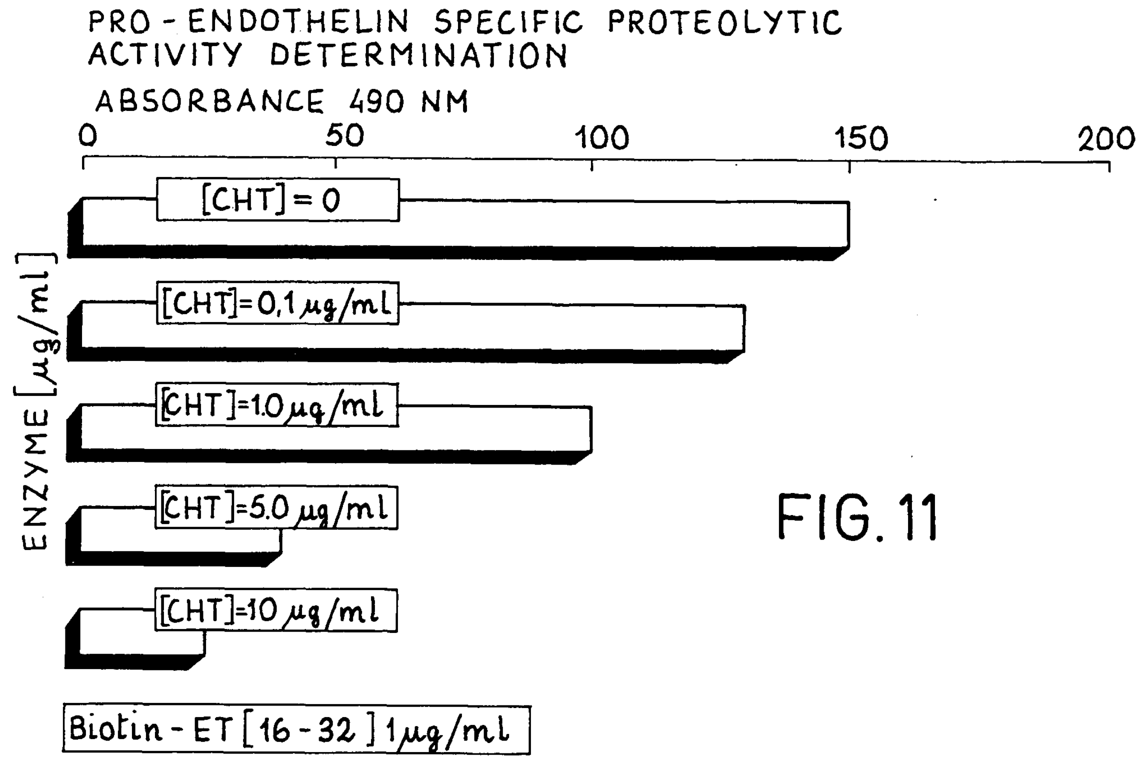
PRO - ENDOTHELIN SPECIFIC PROTEOLYTIC ACTIVITY DETERMINATION
ABSORBANCE 490 NM
0
50
100
150
200
ENZYME [µg/ml]
[CHT] = 0
[CHT]=0,1 µg/ml
[CHT]=1.0 µg/ml
[CHT]=5.0 µg/ml
[CHT]=10 µg/ml
Biotin - ET [16 - 32] 1µg/ml

FIG. 11

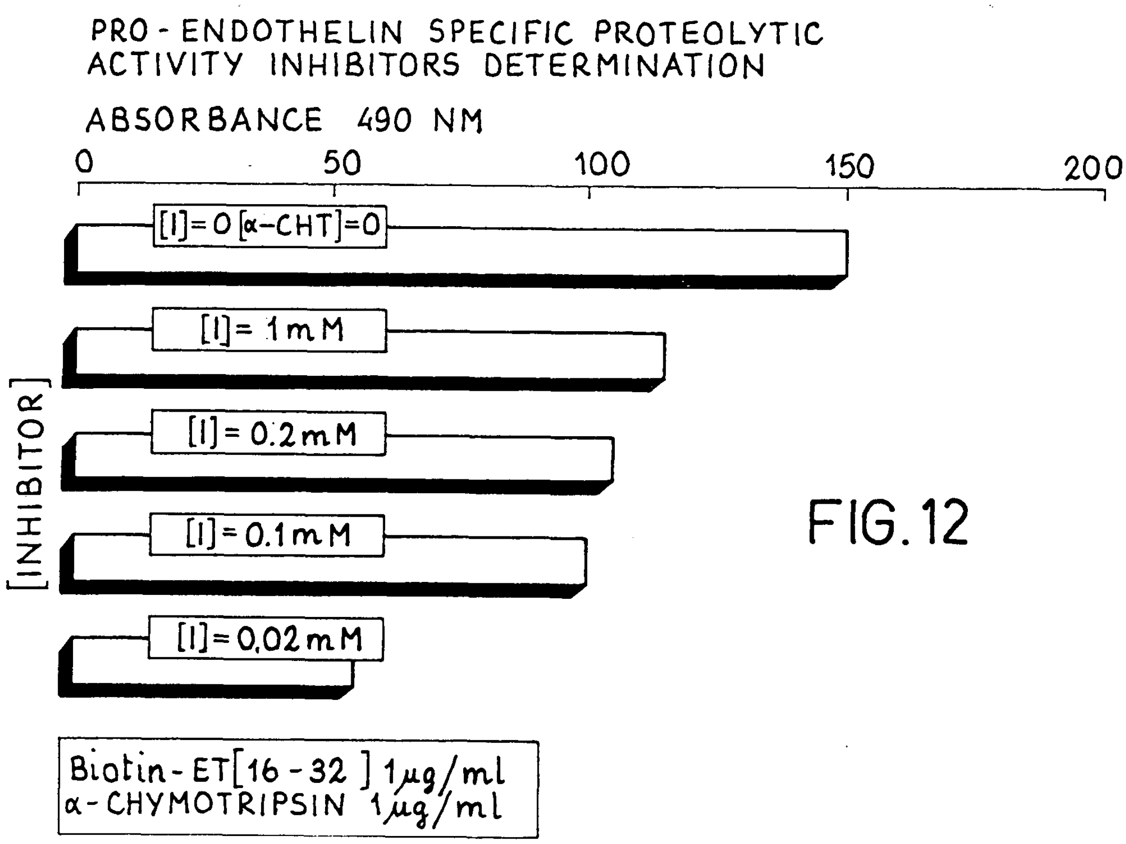
PRO - ENDOTHELIN SPECIFIC PROTEOLYTIC ACTIVITY INHIBITORS DETERMINATION
ABSORBANCE 490 NM
0
50
100
150
200
[INHIBITOR]
[I]= 0 [α-CHT]=0
[I]= 1mM
[I]= 0.2mM
[I]= 0.1mM
[I]= 0.02mM
Biotin - ET[16 - 32] 1µg/ml
α-CHYMOTRIPSIN 1µg/ml

FIG. 12